(19) Europäisches Patentamt
European Patent Office
Office européen des brevets

(11) Veröffentlichungsnummer: **0 202 195**
**A2**

(12) # EUROPÄISCHE PATENTANMELDUNG

(21) Anmeldenummer: **86810206.2**

(22) Anmeldetag: **06.05.86**

(51) Int. Cl.⁴: **C07D 213/64** , //C07C131/00

(30) Priorität: **14.05.85 US 733821**

(43) Veröffentlichungstag der Anmeldung: **20.11.86 Patentblatt 86/47**

(84) Benannte Vertragsstaaten: **BE CH DE FR GB LI NL**

(71) Anmelder: **CIBA-GEIGY AG**
**Klybeckstrasse 141**
**CH-4002 Basel(CH)**

(72) Erfinder: **Rempfler, Hermann, Dr.**
**Brücklismattstrasse 16**
**CH-4107 Ettingen(CH)**
Erfinder: **Pissiotas, Georg, Dr.**
**Am Sonnenrain 71**
**D-7850 Lörrach(DE)**

(54) **Verfahren zur Herstellung von Pyridinyloxy-nitrobenzol-Derivaten.**

(57) Es wird ein neues vierstufiges Verfahren zur Herstellung von herbiziden und pflanzenwuchsregulierenden 2-Nitro-5-(2-pyridinyloxy-acetophenonoximäther-Derivaten der Formel I

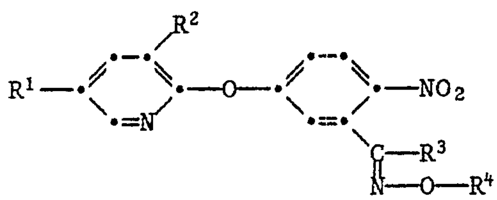

(I)

worin

$R^1$ Wasserstoff, Cyan, Halogen, Nitro, $C_1$-$C_4$-Alkyl oder $C_1$-$C_4$-Halogenalkyl

$R^2$ Wasserstoff oder Halogen,

$R^3$ $C_1$-$C_4$-Alkyl und

$R^4$ $C_1$-$C_4$-Alkyl, $C_1$-$C_4$-Cyanoalkyl, $C_3$-$C_5$-Alkenyl, $C_3$-$C_5$-Halogenalkenyl, $C_3$-$C_5$-Alkinyl, oder durch $C_1$-$C_4$-Alkoxy, Halogen, $C_1$-$C_4$-Alkoxycarbonyl, $C_1$-$C_4$-Alkylaminocarbonyl oder Di-$C_1$-$C_4$-alkylaminocarbonyl substituiertes $C_1$-$C_4$-Alkyl bedeuten,

beschrieben, wobei man ein Phenol der Formel II

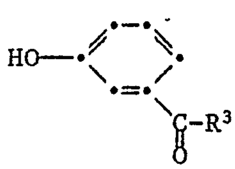

(II)



Rank Xerox

durch Reaktion mit einer Verbindung der Formel III

$H_2N$-O-$R^4$ (III)

in Gegenwart einer Base in einen Oximäther der Formel IV

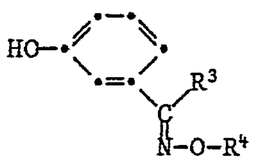

(IV)

überführt; diesen Oximäther in Gegenwart eines säurebindenden Mittels mit einem 2-Halogenpyridin der Formel V

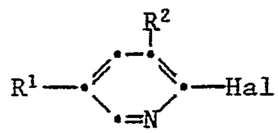

(V)

worin Hal für Chlor oder Brom steht, unsetzt; und den entstandenen Oximäther der Formel VI

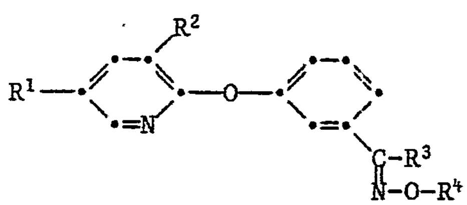

(VI)

mit einem Nitrierungsmittel nitriert.

# Verfahren zur Herstellung von Pyridinyloxy-nitrobenzol-Derivaten

Die vorliegende Erfindung betrifft ein neues Verfahren zur Herstellung von Derivaten der 2-Nitro-5-(2-pyridinyloxy)-acetophenonoximäther, sowie neue Zwischenprodukte, die für dieses Verfahren entwickelt worden sind.

Nach dem erfindungsgemässen Verfahren herstellbare 2-Nitro-5-(2-pyridinyloxy)-acetophenonoximäther-Derivate, ihre Herstellung und Verwendung sind aus dem Europäischen Patent EP-B-23891 bekannt. Diese Wirkstoffe können als selektive Herbizide oder als Pflanzenwuchsregulatoren eingesetzt werden. Das angegebene Herstellungsverfahren erfüllt die Ansprüche an ein Verfahren für die grosstechnische Herstellung der Verbindungen der Formel I; bezüglich der Produktausbeute nur unzureichend. Es besteht daher ein Bedürfnis nach einem Herstellungsverfahren, das das gewünschte Produkt in höherer Ausbeute entstehen lässt. Ueberraschenderweise erfüllt das erfindungsgemässe neue Verfahren diese Anforderung weitgehend.

Erfindungsgemäss wird daher vorgeschlagen, die 2-Nitro-5-(2-pyridinyloxy)-acetophenonoximäther-Derivate der Formel I

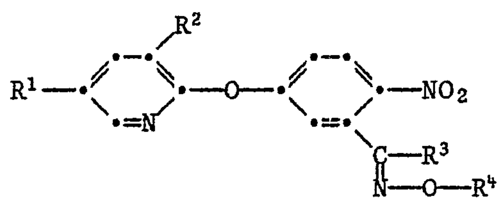

(I)

worin

$R^1$ Wasserstoff, Cyan, Halogen, Nitro, $C_1$-$C_4$-Alkyl oder $C_1$-$C_4$-Halogenalkyl,

$R^2$ Wasserstoff oder Halogen,

$R^3$ $C_1$-$C_4$-Alkyl und

$R^4$ $C_1$-$C_4$-Alkyl, $C_1$-$C_4$-Cyanoalkyl, $C_3$-$C_5$-Alkenyl, $C_3$-$C_5$-Halogenalkenyl, $C_3$-$C_5$-Alkinyl, oder durch $C_1$-$C_4$-Alkoxy, Halogen, $C_1$-$C_4$-Alkoxycarbonyl, $C_1$-$C_4$-Alkylaminocarbonyl oder Di-$C_1$-$C_4$-alkylaminocarbonyl substituiertes $C_1$-$C_4$-Alkyl bedeuten, herzustellen, indem man ein Phenol der Formel II

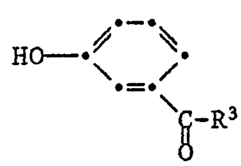

(II)

worin $R^3$ die unter Formel I gegebene Bedeutung hat, durch Reaktion mit einer Verbindung der Formel III

$H_2N$-O-$R^4$ (III)

worin $R^4$ die unter Formel I gegebene Bedeutung hat, in Gegenwart einer Base in einen Oximäther der Formel IV

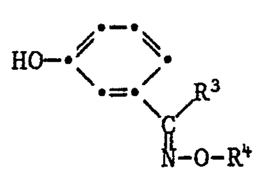

(IV)

worin $R^3$ und $R^4$ die unter Formel I gegebenen Bedeutungen haben, überführt; diesen Oximäther in Gegenwart eines säurebindenden Mittels mit einem 2-Halogenpyridin der Formel V

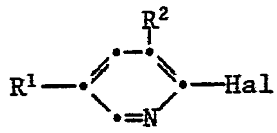

(V)

worin $R^1$ und $R^2$ die unter Formel I gegebenen Bedeutungen haben und Hal für Chlor oder Brom steht, umsetzt; und den entstandenen Oximäther der Formel VI

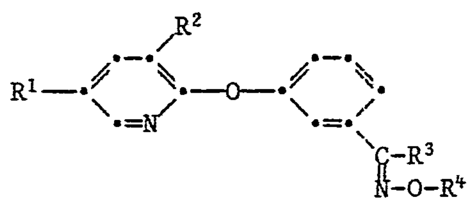

(VI)

worin $R^1$, $R^2$, $R^3$ und $R^4$ die unter Formel I gegebenen Bedeutungen haben, mit einem Nitrierungsmittel nitriert.

In den obigen Definitionen umfassen die angegebenen generischen Begriffe beispielsweise die folgenden spezifischen Einzelsubstituenten, wobei diese Aufzählung keine Einschränkung der Erfindung darstellt:

Alkyl: Methyl, Aethyl, n-Propyl, i-Propyl, n-Butyl, i-Butyl, s-Butyl und t-Butyl, vorzugsweise Methyl und Aethyl.

Halogen: Fluor, Chlor, Brom und Jod, vorzugsweise Fluor und Chlor.

Halogenalkyl: Fluormethyl, Difluormethyl, Trifluormethyl, 2,2,2-Trifluoräthyl, 2-Fluoräthyl, 2-Chloräthyl und 2,2,2-Trichloräthyl, vorzugsweise Trifluoräthyl und Trifluormethyl.

Cyanoalkyl: Cyanomethyl, Cyanoäthyl, Cyanopropyl und Cyanobutyl, vorzugsweise Cyanomethyl.

Alkenyl: Allyl, 2-Butenyl, 3-Butenyl, Methallyl, 2-Methyl-2-butenyl, 3-Methyl-2-butenyl, 3-Pentenyl und 2-Pentenyl, vorzugsweise Allyl und Methallyl.

Alkinyl: Propargyl, 2-Butinyl, 3-Butinyl, 2-Pentinyl, 3-Pentinyl und 4-Pentinyl, vorzugsweise Propargyl.

Halogenalkenyl: 4-Chlor-2-butenyl, 4,4,4,-Trichlor-2-butenyl, 4,4,4-Trifluor-2-butenyl,4-Fluor-2-butenyl.

Weitere unter die Definition von $R^4$ fallende, substituierte Alkylreste sind zum Beispiel: Alkoxyalkyl wie Methoxymethyl, Aethoxymethyl, Methoxyäthyl, Aethoxyäthyl, 2,2-Dimethoxyäthyl, 2,2-Diäthoxyäthyl oder Methoxypropyl, vorzugsweise Methoxyäthyl; die oben aufgezählten Halogenalkylreste; Alkoxycarbonylalkyl wie Methoxycarbonylmethyl, 1-Methoxycarbonyläthyl, 2-Methoxycarbonyläthyl, Aethoxycarbonylmethyl, 1-Aethoxycarbonyläthyl, 1-Aethoxycarbonyläthyl oder i-Propyloxycarbonyläthyl; Alkylaminocarbonylalkyl wie Methylaminocarbonylmethyl, Aethylaminocarbonylmethyl, 1-Methylaminocarbonyläthyl, 2-Methylaminocarbonyläthyl, 1-Aethylaminocarbonyläthyl oder 2-Aethylaminocarbonyläthyl; Dialkylaminocarbonylalkyl wie Dimethylaminocarbonylmethyl, Diäthylaminocarbonylmethyl, 1-Diäthylaminocarbonyläthyl, 2-Diäthylaminocarbonyläthyl, 1-Dimethylaminocarbonyläthyl oder 1-Aethylmethylaminocarbonyläthyl.

Vorzugsweise werden nach dem erfindungsgemässen Verfahren solche Verbindungen der Formel I hergestellt, in denen $R^1$ für Fluor, Chlor, Brom oder Trifluormethyl, $R^2$ für Wasserstoff, Fluor, Chlor oder Brom, $R^3$ für Methyl und $R^4$ für $C_1$-$C_3$-Alkyl oder durch Cyan, $C_1$-$C_3$-Alkoxycarbonyl oder $C_1$-$C_3$-Alkoxy substituiertes $C_1$-$C_3$-Alkyl stehen.

Die Verbindungen der Formel I, ebenso wie die Zwischenprodukte der Formeln IV und VI, können in der Oximätherfunktion in den strukturisomeren E-und Z-Formen anfallen. Ueblicherweise treten Gemische der E-und Z-Form auf, wobei das jeweilige Verhältnis (E:Z), bedingt durch die sterischen Bedingungen der Reaktionspartner, stark schwanken kann. Die Trennung der E/Z-Gemische kann nach üblichen, dem Fachmann geläufigen Trennungsmethoden erfolgen. Soweit es in der vorliegenden Erfindung nicht speziell hervorgehoben wird, sind unter den Verbindungen der Formeln I, IV und VI stets solche E/Z-Gemische gemeint, wie sie durch die erfindungsgemässe Umsetzung erhalten werden. Wo einzelne Strukturisomere gemeint sind, ist dies speziell hervorgehoben.

In der ersten Reaktionsstufe (II + III → IV) wird in an sich bekannter Weise in Gegenwart einer Base die Carbonylgruppe in die Oximäthergruppe überführt. Vorteilhaft ist sowohl die Verwendung eines Lösungsmittels als auch der Zusatz einer Base zum Reaktionsgemisch. Geeignete Lösungsmittel sind: Alkohole wie Methanol, Aethanol oder Isopropanol; chlorierte Kohlenwasserstoffe wie Methylenchlorid, Chloroform, Tetrachlorkohlenstoff, Trichloräthan, Tetrachloräthan, Chlorbenzol oder Dichlorbenzol; Nitrile wie Acetonitril oder Propionitril; Amide wie Dimethylformamid oder N,N-Dimethylacetamid; Ester wie Essigsäureäthylester; oder Dimethylsulfoxid. Als Basen sind sowohl anorganische als auch organische Vertreter anwendbar, wie Alkalihydroxide wie Lithiumhydroxid, Natriumhydroxid oder Kaliumhydroxid; Carbonate wie Natriumcarbonat, Kaliumcarbonat, Magnesiumcarbonat oder Calciumcarbonate; Alkoholate wie Kalium-tert.butylat, Natriummethylat, Natriumäthylat, Kaliumäthylat oder Natriumisopropylat; oder Amine wie Trimethylamin, Triäthylamin, Pyridin, Chinuclidin, 1,4-Diazabicyclo[2,2,2]octan, 1,8-Diazabicyclo[5,4,0]undec-7-en oder 1,5-Diazabicyclo[4,3,0]non-5-en. Die Reaktionstemperaturen liegen im allgemeinen für den ersten Reaktionsschritt (II + III → IV) zwischen 0°C und +120°C, vorzugsweise zwischen +10°C und +50°C. Bevorzugte Reaktionsbedingungen sind für die erste Stufe (II + III → IV): Temperaturen zwischen +10°C und +50°C, die Verwendung von Natriumhydroxid als säurebindendem Mittel und von Aethanol als Lösungsmittel.

In der zweiten Stufe des erfindungsgemässen Verfahrens wird die Aetherkopplung (IV + V → VI) in Gegenwart einer Base durchgeführt, wobei man mit Vorteil ein inertes Lösungsmittel als Reaktionsmedium wählt. Die Reaktionstemperaturen liegen für den zweiten Reaktionsschritt (IV + V → VI) im allgemeinen zwischen -20°C und +120°C, vorzugsweise zwischen 0°C und +60°C.

Geeignete säurebindende Mittel sind:

Alkoholate wie Kalium-tert.butylat, Natriumäthylat, Kaliumäthylat, Natriummethylat oder Natriumisopropylat; Hydroxide wie Lithiumhydroxid, Natriumhydroxid oder Kaliumhydroxid; Oxide wie Magnesium oxid oder Calciumoxid; oder Carbonate wie Natriumcarbonat, Kaliumcarbonat, Magnesiumcarbonat oder Calciumcarbonat. Soll die Reaktion (IV + V → VI) in einem Lösungsmittel durchgeführt werden, verwendet man vorzugsweise ein polares, organisches Lösungsmittel. Als solche sind zu nennen: Ketone wie Aceton, 2-Butanon, Cyclohexanon oder Methylisobutylketon; Nitrile wie Acetonitril oder Propionitril; Amide wie Dimethylformamid oder N,N-Dimethylacetamid; sowie Dimethylsulfoxid. Bevorzugte Reaktionsbedingungen: Temperaturen zwischen 0°C und +60°C, und die Gegenwart von Natriumhydroxid als Base und von Dimethylsulfoxid als Lösungsmittel.

In deitten Reaktionsstufe wird des Zwischenprodukt der Formel VI mit einem Nitrierungsreagenz zur Verbindung der Formel I nitriert. Geeignete übliche Nitrierungsmittel sind: Salpetersäure, Salpetersäure/Schwefelsäure-Gemische, Salpetersäureanhydrid ($N_2O_5$) oder Gemische von Salpetersäure mit Schwefelsäure und Oleum. Die Nitrierungsreaktion kann ohne Lösungsmittel oder mit Vorteil in einem inerten organischen Lösungsmittel durchgeführt werden. Geeignete Lösungsmittel sind: chlorierte Kohlenwasserstoffe wie Methylenchlorid, Chloroform, Tetrachlorkohlenstoff, 1,2-Dichloräthan, Trichloräthan, Tetrachloräthan, Chlorbenzol oder Dichlorbenzol Die Reaktionstemperaturen für die dritte Stufe liegen im allgemeinen zwischen -20°C und +50°C vorzugsweise zwischen -10°C und +30°C. In einer bevorzugten Ausführungsform wird die dritte Stufe (VI → I) bei einer Temperatur zwischen -10°C und +30°C in 1,2-Dichloräthan ausgeführt, wobei als Nitrierungsmittel 100 %-ige Salpetersäure in Gegenwart von Schwefelsäure und Oleum dient.

In einer bevorzugten Ausführungsform des erfindungsgemässen Verfahrens werden die Verbindungen der Formel I erhalten, indem man ein Phenol der Formel II mit einer Verbindung der Formel III bei einer Temperatur zwischen +10°C und +50°C in Gegenwart von Kaliumhydroxid in Aethanol umsetzt, den entstandenen Oximäther der Formel IV bei einer Temperatur zwischen 0°C und +60°C in Gegenwart von Natriumhydroxid in Dimethylsulfoxid mit einem 2-Halogenpyridin der Formel V veräthert; und den entstandenen Oximäther der Formel VI bei einer Temperatur zwischen 0°C und +30°C in 1,2-Dichloräthan mit einem Salpetersäure/Schwefelsäure/Oleum-Gemisch nitriert.

Dieses bevorzugte Verfahren ist insbesondere geeignet, um solche Verbindungen der Formal I herzustellen, in denen $R^1$ für Fluor, Chlor, Brom oder Trifluormethyl, $R^2$ für Wasserstoff, Fluor, Chlor oder Brom, $R^3$ für Methyl und $R^4$ für $C_1$-$C_3$-Alkyl oder durch Cyan, $C_1$-$C_3$-Alkoxycarbonyl oder $C_1$-$C_3$-Alkoxy substituiertes $C_1$-$C_3$-Alkyl stehen.

Die Ausgangsmaterialien der Formeln II, III und V sind bekannt, teilweise im Handel erhältlich und nach literaturbekannten Verfahren herstellbar.

Die Zwischenprodukte der Formeln IV und VI sind neu und wurden speziell für die Durchführung des erfindungsgemässen Verfahrens entwickelt und hergestellt. Die Verbindungen der Formeln IV und VI stellen somit einen Teil der Erfindung dar.

Die folgenden Beispiele dienen der näheren Erläuterung des erfinderischen Verfahrens in seinen einzelnen Verfahrensstufen. Die angeschlossenen Tabellen mit Zwischen-und Endprodukten der Formeln I, IV und VI skizzieren die breite Anwendbarkeit des neuen Verfahrens.

Beispiel 1: 3-Hydroxyacetophenonoximäthyläther

Zu einer Lösung von 13,6 g (0,1 Mol) 3-Hydroxyacetophenon in 200 ml Aethanol werden 9,7 g - (0,1 Mol) Aethoxyamin-hydrochlorid und 10 ml wässrige 30 %-ige Natriumhydroxidlösung zugesetzt. Das Reaktionsgemisch wird für 2 Stunden bei einer Temperatur zwischen 20°C und 25°C gehalten und nach Zusatz von 1 ml Eisessig für 2 Stunden zum Rückfluss erhitzt. Die Lösung wird eingedampft und in einer Mischung von Diäthyläther und Wasser aufgenommen. Die organische Phase wird abgetrennt, mit 10 %-iger Natriumhydrogencarbonatlösung gewaschen, über Natriumsulfat getrocknet und eingedampft. Der Rückstand wird durch Chromatographie an Kieselgel mit einem Essigsäureäthylester/Hexan-Gemisch (1:2) gereinigt. Man erhält 14,9 g (83 % d.Th.) 3-Hydroxyacetophenonoximäthyläther, $n_D^{25}$ 1.5502.

Beispiel 2: 3-(3-Chlor-5-trifluormethylpyridin-2-yloxy)-aceto-phenonoximäthyläther

Eine Lösung von 9,0 g (0,05 Mol) 3-Hydroxyacetophenonoximäthyläther in 50 ml Dimethylsulfoxid wird bei einer Temperatur von 20°C mit 5 ml 20 %-iger wässriger Natriumhydroxidlösung versetzt. Dazu lässt man 10,8 g (0,05 Mol) 2,3-Dichlor-5-trifluormethylpyridin zutropfen. Die Temperatur des Gemisches steigt dabei auf 27°C. Nach einer Reaktionszeit von 2 Stunden nimmt man das Reaktionsgemisch in Eiswasser auf und extrahiert mit Essigsäureäthylester. Die vereinigten organischen Phasen werden mit Wasser gewaschen, über Natriumsulfat getrocknet und eingedampft. Man erhält 17,7 g (99 % d.Th.) 3-(3-Chlor-5-trifluormethylpyridin-2-yloxy)-acetophenonoximäthyläther, $n_D^{25}$ 1.5340.

Beispiel 3: 2-Nitro-5-(3-chlor-5-trifluormethylpyridin-2-yloxy)-acetophenonoximäthyläther

Zu einer Lösung von 5,2 g (0,015 Mol) 3-(3-Chlor-5-trifluormethylpyridin-2-yloxy)-acetophenonoximäthyläther in 50 ml 1,2-Dichloräthan lässt man bei -5°C 10 ml 98 %-ige Schwefelsäure und 1 ml 25 %-iges Oleum zutropfen. Bei 0°C fügt man tropfenweise eine Lösung von 2,5 ml 100 % -iger Salpetersäure in 3 ml 98 %-iger Schwefelsäure zu. Das Reaktionsgemisch wird für 7 Stunden bei Raumtemperatur gerührt, dann mit Eis und Aether versetzt. Die Aetherphase wird mit Wasser und mit einer gesättigten Natriumhydrogencarbonat-Lösung gewaschen, über Natriumsulfat getrocknet und eingedampft. Durch chromatographische Reinigung an Kieselgel mit Essigsäureäthylester/Hexan (1:3) erhält man 3,5 g reinen 2-Nitro-5-(3-chlor-5-trifluormethylpyridin-2-yloxy)-acetophenonoximäthyläther, $n_D^{30}$ 1.5418.

In analoger Weise erhält man die in den Tabellen 1 bis 3 aufgelisteten Zwischen-und Endprodukte.

Tabelle 1:

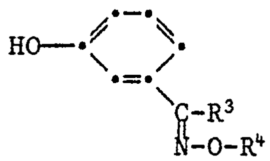

| Verb. Nr. | $R^3$ | $R^4$ | phys. Daten |
|---|---|---|---|
| 1.01 | $CH_3$ | $C_2H_5$ | $n_D^{25}$ 1.5502 |
| 1.02 | $CH_3$ | $CH_3$ | |
| 1.03 | $CH_3$ | $-CH_2-CH=CH_2$ | $n_D^{25}$ 1.5616 |
| 1.04 | $CH_3$ | $-CH_2-CH_2-OCH_3$ | |
| 1.05 | $CH_3$ | $-CH_2-CH_2Cl$ | |
| 1.06 | $CH_3$ | $-CH_2-CH=CHCl$ | |
| 1.07 | $CH_3$ | $-CH_2-COOCH_3$ | |
| 1.08 | $CH_3$ | $-CH_2-COOC_2H_5$ | |
| 1.09 | $CH_3$ | $-CH(CH_3)-COOCH_3$ | |
| 1.10 | $C_2H_5$ | $C_2H_5$ | |
| 1.11 | $C_3H_7$-n | $C_2H_5$ | |

Tabelle 2:

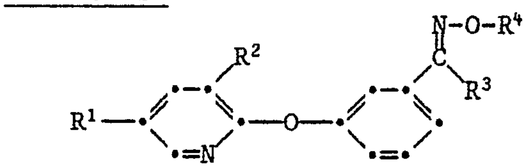

| Verb. Nr. | $R^1$ | $R^2$ | $R^3$ | $R^4$ | Phys. Daten |
|---|---|---|---|---|---|
| 2.01 | Cl | Cl | $CH_3$ | $-CH_2-COOC_2H_5$ | |
| 2.02 | Cl | Cl | $CH_3$ | $-C_2H_5$ | |
| 2.03 | Cl | Cl | $CH_3$ | $-CH(CH_3)-COOCH_3$ | |
| 2.04 | Cl | F | $CH_3$ | $-CH_2-CN$ | |
| 2.05 | Cl | F | $CH_3$ | $-CH_2-COOCH_3$ | |
| 2.06 | $CF_3$ | Cl | $CH_3$ | $-CH_2-COOC_2H_5$ | $n_D^{24}$ 1.5300 |
| 2.07 | $CF_3$ | Cl | $CH_3$ | $-CH_2-CON(CH_3)_2$ | |
| 2.08 | $CF_3$ | Cl | $CH_3$ | $-CH_2-CN$ | |
| 2.09 | $CF_3$ | Cl | $CH_3$ | $-CH_2-CH_2-OCH_3$ | |
| 2.10 | $CF_3$ | Cl | $CH_3$ | $-CH_2-CH_2-OC_2H_5$ | |

Tabelle 2 (Fortsetzung)

| Verb. Nr. | $R^1$ | $R^2$ | $R^3$ | $R^4$ | Phys. Daten |
|---|---|---|---|---|---|
| 2.11 | $CF_3$ | Cl | $CH_3$ | $-CH_2-CH=CH-CH_2Cl$ | |
| 2.12 | $CF_3$ | Cl | $CH_3$ | $-CH_2-CH_2-COOC_2H_5$ | |
| 2.13 | $CF_3$ | Cl | $CH_3$ | $-CH_2-CONHCH_3$ | |
| 2.14 | $CF_3$ | Cl | $CH_3$ | $-CH(CH_3)-COOCH_3$ | |
| 2.15 | $CF_3$ | Cl | $CH_3$ | $-CH(CH_3)-CON(CH_3)_2$ | |
| 2.16 | $CF_3$ | H | $CH_3$ | $-CH_2-COOCH_3$ | |
| 2.17 | $CF_3$ | H | $CH_3$ | $-CH_2-CN$ | |
| 2.18 | $CF_3$ | H | $CH_3$ | $-C_2H_5$ | |
| 2.19 | $CF_3$ | H | $CH_3$ | $-CH_3$ | |
| 2.20 | $CF_3$ | F | $CH_3$ | $-CH_2-COOC_2H_5$ | |
| 2.21 | $CF_3$ | Cl | $C_2H_5$ | $-CH_2-COOC_2H_5$ | |
| 2.22 | $CF_3$ | Cl | $C_3H_7-n$ | $-CH_2-COOC_2H_5$ | |
| 2.23 | $CF_3$ | Cl | $C_4H_9-n$ | $-CH_2-COOC_2H_5$ | |
| 2.24 | $CF_3$ | Cl | $CH_3$ | $CH_3$ | |
| 2.25 | $CF_3$ | Cl | $CH_3$ | $-CH_2-CH=CH_2$ | $n_D^{30}$ 1.5385 |
| 2.26 | $CF_3$ | Cl | $CH_3$ | $-CH_2-CH=CHCl$ | $n_D^{25}$ 1.5619 |
| 2.27 | $CF_3$ | Cl | $CH_3$ | $-CH_2-COOCH_3$ | |
| 2.28 | $CF_3$ | Cl | $C_2H_5$ | $C_2H_5$ | |
| 2.29 | $CF_3$ | Cl | $CH_3$ | $C_2H_5$ | $n_D^{25}$ 1.5340 |
| 2.30 | $CF_3$ | Cl | $C_3H_7-n$ | $C_2H_5$ | |
| 2.31 | Cl | F | $CH_3$ | $-CH_2-CH=CHCl$ | |
| 2.32 | $CF_3$ | F | $CH_3$ | $-CH_2-CH_2-OCH_3$ | |

Tabelle 3:

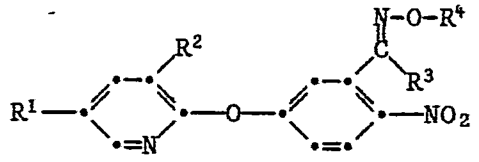

| Verb. Nr. | $R^1$ | $R^2$ | $R^3$ | $R^4$ | Phys. Daten |
|---|---|---|---|---|---|
| 3.01 | Cl | Cl | $CH_3$ | $-CH_2-COOC_2H_5$ | |
| 3.02 | Cl | Cl | $CH_3$ | $-C_2H_5$ | |
| 3.03 | Cl | Cl | $CH_3$ | $-CH(CH_3)-COOCH_3$ | |

Tabelle 3 (Fortsetzung)

| Verb. Nr. | $R^1$ | $R^2$ | $R^3$ | $R^4$ | Phys. Daten |
|---|---|---|---|---|---|
| 3.04 | Cl | F | $CH_3$ | $-CH_2-CN$ | |
| 3.05 | Cl | F | $CH_3$ | $-CH_2-COOCH_3$ | |
| 3.06 | $CF_3$ | Cl | $CH_3$ | $-CH_2-COOC_2H_5$ | $n_D^{25}$ 1.5350 |
| 3.07 | $CF_3$ | Cl | $CH_3$ | $-CH_2-CON(CH_3)_2$ | |
| 3.08 | $CF_3$ | Cl | $CH_3$ | $-CH_2-CN$ | |
| 3.09 | $CF_3$ | Cl | $CH_3$ | $-CH_2-CH_2-OCH_3$ | |
| 3.10 | $CF_3$ | Cl | $CH_3$ | $-CH_2-CH_2-OC_2H_5$ | |
| 3.11 | $CF_3$ | Cl | $CH_3$ | $-CH_2-CH=CH-CH_2Cl$ | |
| 3.12 | $CF_3$ | Cl | $CH_3$ | $-CH_2-CH_2-COOC_2H_5$ | |
| 3.13 | $CF_3$ | Cl | $CH_3$ | $-CH_2-CONHCH_3$ | |
| 3.14 | $CF_3$ | Cl | $CH_3$ | $-CH(CH_3)-COOCH_3$ | $n_D^{35}$ 1.5302 |
| 3.15 | $CF_3$ | Cl | $CH_3$ | $-CH(CH_3)-CON(CH_3)_2$ | |
| 3.16 | $CF_3$ | H | $CH_3$ | $-CH_2-COOCH_3$ | |
| 3.17 | $CF_3$ | H | $CH_3$ | $-CH_2-CN$ | |
| 3.18 | $CF_3$ | H | $CH_3$ | $-C_2H_5$ | |
| 3.19 | $CF_3$ | H | $CH_3$ | $-CH_3$ | |
| 3.20 | $CF_3$ | F | $CH_3$ | $-CH_2-COOC_2H_5$ | |
| 3.21 | $CF_3$ | Cl | $C_2H_5$ | $-CH_2-COOC_2H_5$ | |
| 3.22 | $CF_3$ | Cl | $C_3H_7-n$ | $-CH_2-COOC_2H_5$ | |
| 3.23 | $CF_3$ | Cl | $C_4H_9-n$ | $-CH_2-COOC_2H_5$ | |
| 3.24 | $CF_3$ | Cl | $CH_3$ | $CH_3$ | |
| 3.25 | $CF_3$ | Cl | $CH_3$ | $-CH_2-CH=CH_2$ | |
| 3.26 | $CF_3$ | Cl | $CH_3$ | $-CH_2-CH=CHCl$ | |
| 3.27 | $CF_3$ | Cl | $CH_3$ | $-CH_2-COOCH_3$ | |
| 3.28 | $CF_3$ | Cl | $C_2H_5$ | $C_2H_5$ | |
| 3.29 | $CF_3$ | Cl | $CH_3$ | $C_2H_5$ | $n_D^{30}$ 1.5418 |
| 3.30 | $CF_3$ | Cl | $C_3H_7-n$ | $C_2H_5$ | |
| 3.31 | Cl | F | $CH_3$ | $-CH_2-CH=CHCl$ | |
| 3.32 | $CF_3$ | F | $CH_3$ | $-CH_2-CH_2-OCH_3$ | |

## Ansprüche

1. Verfahren zur Herstellung von 2-Nitro-5-(2-pyridinyloxy)acetophenonoximäther-Derivaten der Formel I

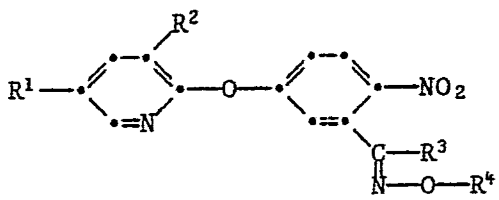

(I)

worin

$R^1$ Wasserstoff, Cyan, Halogen, Nitro, $C_1$-$C_4$-Alkyl oder $C_1$-$C_4$-Halogenalkyl,

$R^2$ Wasserstoff oder Halogen,

$R^3$ $C_1$-$C_4$-Alkyl und

$R^4$ $C_1$-$C_4$-Alkyl, $C_1$-$C_4$-Cyanoalkyl, $C_3$-$C_5$-Alkenyl, $C_3$-$C_5$-Halogenalkenyl, $C_3$-$C_5$-Alkinyl, oder durch $C_1$-$C_4$-Alkoxy, Halogen, $C_1$-$C_4$-Alkoxycarbonyl, $C_1$-$C_4$-ALkylaminocarbonyl oder Di-$C_1$-$C_4$-alkylaminocarbonyl substituiertes $C_1$-$C_4$-Alkyl bedeuten, dadurch gekennzeichnet, dass man ein Phenol der Formel II

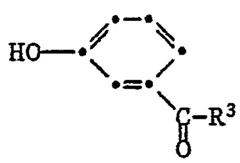

(II)

worin $R^3$ die unter Formel I gegebene Bedeutung hat, durch Reaktion mit einer Verbindung der Formel III

$H_2N$-O-$R^4$ (III)

worin $R^4$ die unter Formel I gegebene Bedeutung hat, in Gegenwart einer Base in einen Oximäther der Formel IV

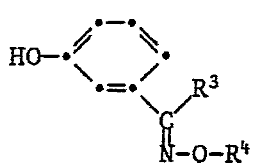

(IV)

worin $R^3$ und $R^4$ die unter Formel I gegebenen Bedeutungen haben, überführt; diesen Oximäther in Gegenwart eines säurebindenden Mittels mit einem 2-Halogenpyridin der Formel V

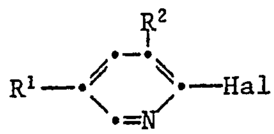

(V)

worin $R^1$ und $R^2$ die unter Formel I gegebenen Bedeutungen haben und Hal für Chlor oder Brom steht, umsetzt; und den entstandenen Oximäther der Formel VI

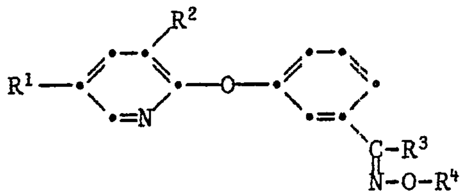

(VI)

worin $R^1$, $R^2$, $R^3$ und $R^4$ die unter Formel I gegebenen Bedeutungen haben, mit einem Nitrierungsmittel nitriert.

2. Verfahren gemäss Anspruch 1, dadurch gekennzeichnet, dass $R^1$ für Fluor, Chlor, Brom oder Trifluormethyl, $R^2$ für Wasserstoff, Fluor, Chlor oder Brom, $R^3$ für Methyl und $R^4$ für $C_1$-$C_3$-Alkyl oder durch Cyan, $C_1$-$C_3$-Alkoxycarbonyl oder $C_1$-$C_3$-Alkoxy substituiertes $C_1$-$C_3$-Alkyl stehen.

3. Verfahren gemäss Anspruch 1, dadurch gekennzeichnet, dass man die Umsetzung der Verbindung II mit der Verbindung III in Gegenwart von Natriumhydroxid in Aethanol bei einer Temperatur zwischen +10°C und +50°C durchgeführt.

4. Verfahren gemäss Anspruch 1, dadurch gekennzeichnet, dass man die Umsetzung des Oximäthers der Formel IV mit dem 2-Halogenpyridin der Formel V bei Temperaturen zwischen 0°C und +60°C in Gegenwart von Natriumhydroxid in Aethanol durchführt.

5. Verfahren gemäss Anspruch 1, dadurch gekennzeichnet, dass man den Oximäther der Formel VI bei einer Temperatur zwischen -10°C und +30°C in 1,2-Dichloräthan mit einem Salpetersäure/Schwefelsäure/Oleium-Gemisch nitriert.

6. Verfahren gemäss Anspruch 1, dadurch gekennzeichnet, dass man ein Phenol der Formel II mit einer Verbindung der Formel III bei einer Temperatur zwischen +10°C und +50°C in Gegenwart von Kaliumhydroxid in Aethanol umsetzt, den entstandenen Oximäther der Formel IV bei einer Temperatur zwischen 0°C und +60°C in Gegenwart von Natriumhydroxid in Dimethylsulfoxid mit einem 2-Halogenpyridin der Formel V veräthert; und den entstandenen Oximäther der Formel VI bei einer Temperatur zwischen -10°C und +30°C in 1,2-Dichloräthan mit einem Salpetersäure/Schwefelsäure/Oleum-Gemisch nitriert.

7. Verfahren gemäss Anspruch 1, dadurch gekennzeichnet, dass man Verbindungen der Formel I herstellt, worin $R^1$ für Fluor, Chlor, Brom oder Trifluormethyl, $R^2$ für Wasserstoff,Fluor, Chlor oder Brom, $R^3$ für Methyl und $R^4$ für $C_1C_3$-Alkyl oder durch Cyan, $C_1$-$C_3$-Alkoxycarbonyl oder $C_1$-$C_3$-Alkoxy substituiertes $C_1$-$C_3$-Alkyl stehen.

8. Oximäther der Formel IV

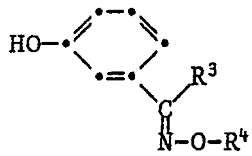

(IV)

worin

$R^3$ $C_1$-$C_4$-Alkyl und

$R^4$ $C_1$-$C_4$-Alkyl, $C_1$-$C_4$-Cyanoalkyl, $C_3$-$C_5$-Alkenyl, $C_3$-$C_5$-Halogenalkenyl, $C_3$-$C_5$-Alkinyl, oder durch $C_1$-$C_4$-Alkoxy, Halogen, $C_1$-$C_4$-Alkoxycarbonyl, $C_1$-$C_4$-Alkylaminocarbonyl oder Di-$C_1$-$C_4$-alkylaminocarbonyl substituiertes $C_1$-$C_4$-Alkyl bedeuten.

9. 3-Hydroxyacetophenonoximäthyläther gemäss Anspruch 8.

10. 3-Hydroxyacetophenonoximallyläther gemäss Anspruch 8.